(19) Europäisches Patentamt
European Patent Office
Office européen des brevets

(11) **EP 4 635 981 A1**

(12) **EUROPEAN PATENT APPLICATION**
published in accordance with Art. 153(4) EPC

(43) Date of publication:
**22.10.2025 Bulletin 2025/43**

(21) Application number: **22968338.8**

(22) Date of filing: **30.12.2022**

(51) International Patent Classification (IPC):
*C07K 16/28* (2006.01)       *A61K 47/68* (2017.01)
*A61K 49/00* (2006.01)       *A61K 51/10* (2006.01)
*C12N 15/13* (2006.01)       *C12N 15/85* (2006.01)
*G01N 33/574* (2006.01)      *C12N 5/10* (2006.01)

(52) Cooperative Patent Classification (CPC):
A61K 47/68; A61K 49/00; A61K 51/10;
C07K 16/00; C07K 16/28; C12N 5/10; C12N 15/85;
G01N 33/574

(86) International application number:
**PCT/CN2022/143892**

(87) International publication number:
**WO 2024/124637 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.12.2022  CN 202211622638**

(71) Applicant: **Carbiogene Therapeutics Co., Ltd.
Hangzhou, Zhejiang 310051 (CN)**

(72) Inventors:
• **YANG, Xin
  Hangzhou, Zhejiang 310051 (CN)**

• **ZHU, Jiangao
  Hangzhou, Zhejiang 310051 (CN)**
• **YANG, Wenjun
  Hangzhou, Zhejiang 310051 (CN)**
• **YU, Jiandong
  Hangzhou, Zhejiang 310051 (CN)**

(74) Representative: **Frick, Robert
  Lorenz Seidler Gossel
  Rechtsanwälte Patentanwälte
  Partnerschaft mbB
  Widenmayerstraße 23
  80538 München (DE)**

Remarks:
The complete document including Reference
Table(s) and the Sequence Listing(s) can be
downloaded from the EPO website

(54) **ANTI-CLL1 SINGLE-DOMAIN ANTIBODY AND USE THEREOF**

(57)    The present invention relates to an anti-CLL1 single-domain antibody and use thereof, specifically, to a single-domain antibody having an amino acid sequence of SEQ ID No. 1. The single-domain antibody has high affinity and can specifically target CLL1-positive cells, and can be applied to the detection of CLL1 expression in bone marrow cells of acute myeloid leukemia (AML) patients. The single-domain antibody can be prepared into a specific antibody drug clinically used for preventing and treating CLL1 target-related diseases (such as acute myeloid leukemia, myelodysplastic syndromes, or chronic myeloid leukemia), and can also be used in the preparation of CLL1-targeting chimeric antigen receptor (CAR) cells or diagnostic kits for CLL1 protein detection and the like. The single-domain antibody drug has a stable structure, small molecular size, ease of recombinant expression, and low production cost. It can be used alone or employed as a drug delivery system to carry related drugs, which has broad prospects and important significance in the fields of pharmaceutical application, clinical diagnosis, and related fields.

## Description

### Technical Field

The present invention belongs to the field of biomedicine, and specifically to anti-CLL1 single-domain antibodies and use thereof.

### Background Art

Acute myeloid leukemia (AML) is a malignant disease of myeloid hematopoietic stem/progenitor cells. For a long time, anthracycline and cytarabine chemotherapies have been used to treat low-risk patients, and hematopoietic stem cell transplantation has been used to treat medium and high-risk patients. The heterogeneity of acute myeloid leukemia still poses significant challenges in treatment. Recent studies have shown that CLL1-targeted immunotherapy is highly effective against AML. C-type lectin-like molecule 1 (CLL1), also known as C-type lectin domain family 12 member A (CLEC12A) , is a type II transmembrane glycoprotein that plays an important role as an inhibitory receptor in immune regulation. CLL1 is expressed on myeloid cells in peripheral blood and bone marrow, as well as on most AML cells , including AML $CD34^+CD38^-$ stem cells in AML patients, but not on normal $CD34^+CD38^-$ stem cells. Due to its unique expression pattern, CLL1 has become a potential target for AML treatment and diagnosis. In addition,CLL1 is also expressed on cells of myelodysplastic syndromes (MDS) and chronic myeloid leukemia (CML).

Antibody (Ab) is an effector immune molecule produced by the proliferation and differentiation of B cells into plasma cells after being specifically stimulated by B cell antigen epitopes. It mediates humoral immunity and is a type of immunoglobulin (Ig) that can specifically bind to an antigen. Ig specifically recognizes and binds to the corresponding antigen epitope in a lock-key complementary relationship through an antigen binding slot composed of its V region CDRs. The specific binding capacity of antibodies to antigens makes them of great significance in disease diagnosis and immune prevention and treatment. Antibody drugs composed of antibody substances (including whole antibody molecules and antibody fragments with therapeutic functions) are one of the important means of targeted therapy and have become the most promising and valuable hot topics in the current biopharmaceutical industry.

Heavy chain antibody (HcAb) is a naturally occurring type of antibody that is an antibody type unique in camelids or cartilaginous fish. Its antibody domain naturally lacks a light chain and is composed of only two heavy chains. The antigen recognition function of heavy chain antibodies is mainly determined by variable region (VHH) of heavy chain antibodies. VHH alone can recognize antigens, hence it is also known as single-domain antibody (sdAb). Single-domain antibody lacks an antibody light chain and only has the heavy chain variable region. Due to its small molecular weight, it is also known as nanobody (Nb). Single-domain antibody has molecular weight of only about 13-15 KDa, a diameter of about 2.5 nm and a length of 4 nm, which is the smallest antibody fragment with antigen binding function to date. The ability and stability of a single-domain antibody to bind to an antigen is basically the same as that of a complete antibody or has higher specific antigen affinity. Compared with traditional antibodies, single-domain antibodies also have many unique properties, such as good stability, the ability to reach specific antigen epitopes, the ability to combine block patterns arbitrarily, and low production costs. The conventional methods for obtaining single-domain antibodies consist of numerous steps such as multiple immunizations of camelids, isolation of B lymphocytes, amplification of VHH regions, construction of display libraries, screening and so on. With the development of synthetic biology, it has become possible to construct high-quality randomized large capacity single-domain antibody libraries based on total synthesis. At present, VHH single-domain antibodies have been widely used in small-scale genetic engineering antibody research, new drug development, and disease diagnosis and treatment due to their stable structure, small molecule size, good solubility, tolerance to various adverse environments, good formulation stability, easy recombinant expression, and easy humanization and so on. The research and development of single-domain antibodies have broad prospects and significant implications in fields such as drug applications and clinical diagnosis.

### Summary of the Invention

One of the objectives of the present invention is to provide a single-domain antibody targeting CLL1 antigen and its use in tumor targeted therapy.

To achieve the above objective, the present invention first provides a single-domain antibody (anti-CLL1 single-domain antibody), named as CLL1-VHH-16, which is composed of a heavy chain variable region (VHH). The heavy chain variable region may comprise CDR1 having an amino acid sequence of positions 26-35 of SEQ ID No.1, CDR2 having an amino acid of positions 50-59 of SEQ ID No.1, and CDR3 having an amino acid of positions 99-118 of SEQ ID No.1.

The single-domain antibody (anti-CLL1 single domain antibody) can be a single domain antibody that specifically binds to CLL1 protein.

Further, the amino acid sequence of the variable region of the heavy chain can be SEQ ID No.1 or an amino acid sequence that is at least 80% identical to SEQ ID No.1 and has the same function. The single-domain antibody (anti-CLL1 single-domain antibody) only has the variable region (VHH) of the heavy chain antibody, which is composed of a framework region FR1, complementarity determining region CDR1, a framework region FR2, complementarity determining region CDR2, a framework region FR3, complementarity determining region CDR3, and a framework region FR4 in sequence.

The amino acid sequence of the single-domain antibody CLL1-VHH-16 can be SEQ ID No.1. Wherein, positions 1-25 of SEQ ID No.1 are the frame region FR1, positions 26-35 of SEQ ID No.1 are the complementary determining region CDR1, positions 36-49 of SEQ ID No.1 are the frame region FR2, positions 50-59 of SEQ ID No.1 are the complementary determining region CDR2, positions 60-98 of SEQ ID No.1 are the frame region FR3, positions 99-118 of SEQ ID No.1 are the complementary determining region CDR3, and positions 119-129 of SEQ ID No.1 are the frame region FR4. A nucleotide sequence of a nucleic acid molecule encoding the single-domain antibody CLL1-VHH-16 (CLL1-VHH-16 gene) is shown in SEQ ID No.2.

The sequence of the complementary determining region is defined according to Kabat numbering system.

Other variants of the single-domain antibody sequence of the present invention with improved affinity and/or valence can be obtained by using methods known in the art and are included within the protection scope of the present invention. For example, amino acid substitution can be used to obtain antibodies with further improved affinity. Alternatively, codon optimization of nucleotide sequences can also be used to improve translation efficiency in expression systems used for antibody production. In addition, polynucleotides comprising sequences optimized for antibody specificity by applying directed evolution to any nucleic acid sequence of the present invention are also within the scope of the present invention.

In certain embodiments, substitution, insertion, or deletion may occur within one or more complementary determining regions or framework regions of the single-domain antibody of the present invention, as long as such change(s) does not substantially reduce antibody's ability to bind to antigens. For example, conservative changes can be made to a complementary determining region and/or a framework region (such as conservative substitution, as is well known to those skilled in the art, a conservative substitution of an amino acid does not alter the properties and functions of a protein), without substantially reducing binding affinity. For example, such change(s) can occur outside of antigen contacting residues in a complementarity determining region.

The present invention also provides a biomaterial related to the single-domain antibody CLL1-VHH-16, which can be any one of C1) to C4):

C1) a nucleic acid molecule encoding the heavy chain variable region of the single-domain antibody CLL1-VHH-16; C2) an expression cassette containing the nucleic acid molecule of C1); C3) a recombinant vector containing the nucleic acid molecule of C1), or a recombinant vector containing the expression cassette of C2); C4) a recombinant host cell containing the nucleic acid molecule of C1), or a recombinant host cell containing the expression cassette of C2), or a recombinant host cell containing the recombinant vector of C3).

Among them, the recombinant host cell described in C4) can express the single-domain antibody CLL1-VHH-16.

In the above-mentioned biological material, the nucleic acid molecule can be any one of the following:

D1) a DNA molecule having a nucleotide sequence or coding sequence of SEQ ID No.2; D2) a DNA molecule that has 75% or more identity with the nucleotide sequence defined by D1) and has the same function.

The DNA molecule shown in SEQ ID No.2 encodes the single-domain antibody CLL1-VHH-16 having amino acid sequence of SEQ ID No.1.

The above 75% or more identity can be 80%, 85%, 90%, or 95% or more identity.

The above 80% or more identity can be at least 80%, 81%, 82%, 83%, 84%, 85%, 86%, 87%, 88%, 89%, 90%, 91%, 92%, 93%, 94%, 95%, 96%, 97%, 98% or 99% identity. The above 85% or more identity can be at least 85%, 86%, 87%, 88%, 89%, 90%, 91%, 92%, 93%, 94%, 95%, 96%, 97%, 98% or 99% identity. The 90% or more identity can be at least 90%, 91%, 92%, 93%, 94%, 95%, 96%, 97%, 98% or 99% identity. The 95% or more identity can be at least 95%, 96%, 97%, 98%, or 99% identity.

Herein, identity refers to the identity of amino acid sequences or nucleotide sequences. The identity of amino acid sequences can be determined using homology search on the Internet, such as the BLAST webpage on the NCBI website. For example, in advanced BLAST2.1, by using blastp as a program, setting Expect value to 10 and all Filters to OFF, using BLOSUM62 as Matrix, and setting Gap existence cost, Per residue gap cost, and Lambda ratio to 11, 1, and 0.85 (default values), respectively, and preforming retrieval to calculate the identity of the amino acid sequence. Then, a value of identity (%) can be obtained.

The vector recited herein refers to a vector that can carry an exogenous DNA or a target gene into host cells for amplification and expression. The vector can be a cloning vector or an expression vector, including but not limited to: plasmids, phages (such as lambda phages or M13 filamentous phages), cosmids (i.e. Kos plasmids), artificial chromosomes (such as yeast artificial chromosomes (YAC), bacterial artificial chromosomes (BAC), P1 artificial chromosome (PAC) or Ti plasmid artificial chromosomes (TAC), etc.), viral vectors (such as baculovirus vectors, retroviruses (including lentiviruses), adenovirus, adeno-associated virus or herpes simplex virus (such as herpes simplex virus), etc.). In one or more embodiments of the present invention, the vector is a pComb3XSS plasmid and/or a pcDNA3.1 vector.

The host cell (also known as receptient cell) recited herein refers to any type of cell that can be used for vector introduction. Host cells can be eukaryotic cells (such as plant cells, animal cells, fungi, or algae), or prokaryotic cells (such as bacteria or protozoa). The host cell can be understood as referring not only to specific receptient cells, but also to progeny of such cells, and due to natural, accidental, or intentional mutations and/or changes, the progeny may not necessarily be completely identical to the original parent cells, but still fall within the scope of the host cell. Suitable host cells are known in this art, wherein, the plant cells may be plant cells such *Arabidopsis*(*Arabidopsis thaliana*), tobacco (*Nicotiana tabacum*), corn (*Zea mays*), rice (*Oryza sativa*), and wheat (*Triticum aestivum*), but not limited thereto; the animal cells can be mammalian cells (such as Chinese hamster ovary cells (CHO cells), African green monkey kidney cells (Vero cells), baby hamster kidney cells (BHK cells), mouse breast cancer cells (C127 cells), human embryonic kidney cells (HEK293 cells), human HeLa cells, fibroblasts, bone marrow cell lines, T cells or NK cells, etc.), poultry cells (such as chicken or duck cells), amphibious cells (such as African clawed frog (*Xenopus laevis*) cells or *Andrias davidianus* cells), fish cells (such as Ctenopharyngodon, carp, rainbow trout or catfish cells), insect cells (such as Sf21 cells or Sf-9 cells), etc., but not limited thereto. The fungus may be yeast, which can be from saccharomyces (such as *Saccharomyces cerevisiae*), Kluyveromyces genus (such as *Kluyveromyces lactis*), Pichia genus (such as *Pichia pastoris*), Schizosaccharomyces genus (such as *Schizosaccharomyces pombe*), Hansenula genus (such as *Hansenula polymorpha*), etc., but not limited to thereto. The fungi can also from the genera of Fusarium (*Fusarium sp.*), Rhizoctonia (*Rhizoctonia sp.*), Verticillium (*Verticillium sp.*), Penicillium (*Penicillium sp.*), Aspergillus (*Aspergillus sp.*), *Cephalosporium sp.,* etc., but are not limited thereto. The algae may be from the genera of Fucus (*Fucus sp.*), Achnanthes (*Achnanthes sp.*), *Amphiphora sp., Amphora sp., Ankistodemus sp., Asteromonas sp., Boekelovia sp.,* etc., but are not limited thereto. Bacteria can from the genera of *Escherichia sp., Erwinia sp., Agrobacterium sp., Flavobacterium sp., Alcaligenes sp., Pseudomonas sp., Bacillus sp.,* etc., but are not limited thereto. For example, the bacteria may be *Escherichia coli, Bacillus subtilis,* or *Bacillus pumilus.* In one or more embodiments of the present invention, the host cell is *Escherichia coli* TG1 and/or HEK293 cells.

The recombinant vector recited herein refers to a recombinant DNA molecule constructed by connecting an exogenous target gene with a vector in vitro. It can be constructed in any suitable way, as long as the constructed recombinant vector can carry an exogenous target gene and enter into a recipient cell and provide ability for replication, integration, amplification, and/or expression of the exogenous target gene in the recipient cell. In one or more embodiments of the present invention, the recombinant vector is pcDNA3.1-CLL1-VHH-16-hFc.

The recombinant vector pcDNA3.1-CLL1-VHH-16-hFc is the recombinant eukaryotic expression vector pcDNA3.1-CLL1-VHH-16-hFc of the single-domain antibody CLL1-VHH-16 for expressing hFc gene fused at C- terminus, which is obtained by: adding human derived IgG1Fc gene (hFc gene, nucleotide sequence of which is SEQ ID No. 3) to 3 'end of the coding gene (SEQ ID No.2) of the single-domain antibody CLL1-VH-16 to obtain a CLL1-VHH16-hFc fusion gene; then adding a *BamHI* enzyme cleavage site to be before the start codon of 5 'end of the fusion gene, and an *EcoRI* enzyme cleavage site to be after the stop codon of 3 'end of the fusion gene, respectively; and cloning it into an eukaryotic expression vector pcDNA3.1 between the *BamHI* and *EcoRI* recognition sites to obtain a recombinant eukaryotic expression vector pcDNA3.1-CLL1-VHH-16-hFc expressing the single-domain antibody CLL1-VHH-16 fused with hFc gene at C-terminal. The recombinant eukaryotic expression vector pcDNA3.1-CLL1-VHH-16-hFc contains the coding gene for the single-domain antibody CLL1-VHH-16 shown in SEQ ID No. 2. The nucleotide sequence (696 bp) of the human derived IgG1Fc gene is shown in SEQ ID No.3, and its encoded amino acid sequence is shown in SEQ ID No.4.

The recombinant host cell recited herein refers to a functionally altered recombinant host cell obtained by manipulating and modifying the genes of a target host cell, such as recombinant host cells obtained by introducing exogenous target gene(s) or recombinant vector(s) into target host cell(s), or recombinant host cells obtained by directly editing endogenous gene(s) of target host cells. The recombinant host cell can be understood not only as referring to a specific recombinant host cell, but also as to progeny of such cells, and due to natural, accidental, or intentional mutations and/or changes, the progeny may not necessarily be completely identical to an original parent cell, but still fall within the scope of recombinant host cells. In one or more embodiments of the present invention, the recombinant host cell is a recombinant cell obtained by introducing the recombinant vector pcDNA3.1-CLL1-VHH-16-hFc into HEK293 cells.

The present invention also provides a pharmaceutical composition, which may comprise the single-domain antibody CLL1-VHH-16.

Furthermore, the pharmaceutical composition may further comprise a pharmaceutically acceptable carrier.

The pharmaceutically acceptable carriers can be diluents, excipients, fillers, adhesives, wetting agents, disintegrants, absorption enhancers, adsorption carriers, surfactants, or lubricants.

The pharmaceutical composition has at least one of the following uses:

M1) use in prevention or treatment of tumors;
M2) use in prevention or treatment of CLL1 target related diseases;
M3) use in mediating drug specific recognition of a tumor expressing CLL1 antigen.

The present invention also provides an agent or kit, which may contain the single-domain antibody CLL1-VHH-16. The agent or kit has at least one of the following uses:

F1) detection of CLL1 protein;
F2) screening, diagnosis, or auxiliary diagnosis of CLL1 target related diseases;
F3) use for *in vivo* imaging of CLL1 protein.

The kit can be a chemiluminescence immunoassay kit, an enzyme-linked immunosorbent assay kit, a colloidal gold immunoassay kit, or a fluorescent immunoassay kit, but is not limited thereto. The present invention also provides any one of uses of the single-domain antibody CLL1-VHH-16 and/or the biomaterial:

E1) use in the preparation of a medicament for prevention or treatment of a tumor, or in prevention or treatment of a tumor;
E2) use in the preparation of a medicament for prevention or treatment of a CLL1 target related disease, or in prevention or treatment of a CLL1 target related disease;
E3) use in the preparation of a product for screening, diagnosing, or assisting in the diagnosis of CLL1 target related diseases, or in the screening, diagnosis, or assisting in the diagnosis of a CLL1 target related disease;
E4) use in detecting CLL1 protein or in preparing a product for detecting CLL1 protein;
E5) use in the preparation of a product for binding to CLL1 protein;
E6) use in mediating drug specific recognition of a tumor expressing CLL1 antigen or in preparing a product that mediates drug specific recognition of a tumor expressing CLL1 antigen;
E7) use in *in vivo* imaging of CLL1 protein or in the preparation of a product for *in vivo* imaging of CLL1 protein;
E8) use in the preparation of CAR cells targeting CLL1.

Herein, the CLL1 target related disease can be a CLL1 positive cancer (such as leukemia).
Herein, the CLL1 positive cancer may be acute myeloid leukemia (AML), myelodysplastic syndromes (MDS), and chronic myeloid leukemia (CML), but are not limited thereto.
The product that mediates drug specific recognition of a tumor expressing CLL1 antigen as described in E6) can be a drug delivery system that specifically targets CLL1 protein, comprising the single-domain antibody CLL1-VHH-16.
The drug delivery system can be a liposome drug delivery system, a polymer micelle drug delivery system, a polymer disc drug delivery system, or a nanoparticle drug delivery system.
The drug delivery system can be used for targeted transport and/or targeted release of drugs.
The CAR cell targeting CLL1 as described in E8) expresses a chimeric antigen receptor (CARs), which may contain the single-domain antibody CLL1-VHH-16.
Furthermore, an antigen recognition domain of the chimeric antigen receptor can be the single-domain antibody CLL1-VHH-16.
Furthermore, the CAR cell includes but not limited to CAR-T cell, CAR-NK cell, CAR macrophage (CAR-M cell), CAR iPSC, or CAR-PSC.
The detection of CLL1 protein described herein can be detecting whether a sample to tested contains CLL1 protein and/or detecting the content of CLL1 protein in a sample to be tested.
The sample to be tested can be a cell or a tissue sample.
The product for detecting CLL1 protein includes a product that detects binding of antigen and antibody using enzyme-linked immunosorbent assay, immunofluorescence detection, radioimmunoassay, luminescence immunoassay, colloidal gold immunochromatography, agglutination assay, or immunoturbidimetry.
The products described herein can be reagents, kits, chips, or test strips.
The present invention also provides a method for preparing the single-domain antibody CLL1-VHH-16, the method may include: constructing a recombinant expression vector containing a nucleic acid molecule encoding the heavy chain variable region of the single-domain antibody CLL1-VHH-16; introducing the recombinant expression vector into host cells to obtain recombinant cells expressing the single-domain antibody; culturing the recombinant cells and obtaining the single-domain antibody through isolation and purification.
Furthermore, the nucleic acid molecule encoding the heavy chain variable region of the single-domain antibody CLL1-VHH-16 can be any one of the following:

D1) a DNA molecule having a nucleotide sequence or a coding sequence of SEQ ID No.2;

D2) a DNA molecule that has 75% or more identity with the nucleotide sequence defined by D1) and has the same function.

Further, the host cell can be HEK293 cell.

Further, the introducing can be achieved by transforming a vector carrying the single-domain antibody gene of the present invention into host cells by any known transfection method, such as calcium phosphate co-precipitation, liposome mediated transformation, electroporation, or viral vector method.

The present invention also provides a method for diagnosing or assisting in diagnosis of a CLL1 target related disease, the method may include: isolating a sample from a subject, and then detecting the content of CLL1 protein in the sample using the single-domain antibody CLL1-VHH-16, and diagnosing or assisting in diagnosis of CLL1 target related disease based on the content of CLL1 protein (compared with the content of CLL1 protein in disease diagnostic criteria). The present invention also provides a method for screening CLL1 target related diseases, the method may include: isolating a sample from a subject, and then detecting the content of CLL1 protein in the sample using the single-domain antibody CLL1-VHH-16, and screening for CLL1 target related diseases based on the content of CLL1 protein (compared with the content of CLL1 protein in disease screening standards).

The present invention also provides a method for preventing or treating a CLL1 target related disease, the method may include administering the single-domain antibody CLL1-VHH-16 or the pharmaceutical composition described herein to a subject suffering from CLL1 target related disease.

In the above method, the CLL1 target related disease may be a CLL1 target related tumor.

In the above method, the CLL1 target related tumor can be a CLL1 positive cancer.

In the above method, the CLL1 positive cancer can be acute myeloid leukemia, myelodysplastic syndrome, or chronic myeloid leukemia.

The present invention also provides a method for *in vitro* detection of CLL1 protein, which may include detecting CLL1 protein using the single-domain antibody CLL1-VHH-16 or the kit described herein.

The present invention also provides antibody drug conjugates, comprising an antibody portion and a conjugate portion, wherein the antibody portion may comprise the single-domain antibody CLL1-VHH-16.

Further, the conjugate portion may include detectable markers, chemical drugs, or cytotoxins.

The antibody portion and the conjugate portion can be covalently linked directly or through a linker.

The detectable markers can be selected from enzymes (such as horseradish peroxidase or alkaline phosphatase), chemiluminescence reagents (such as acridine ester compounds, luminol and its derivatives, or ruthenium derivatives), fluorescent dyes (such as fluorescein or fluorescent proteins), radioactive isotopes, or biotin.

The chemical drug may be a drug that can kill tumor cells.

The cytotoxins can be tubulin inhibitors (such as maytansine, auristatin), DNA synthesis inhibitors (such as calicheamicin) or RNA synthesis inhibitors (such as amanita toxin).

The purposes of the use and method of the present invention may be for diagnosis of a disease, disease prognosis, and/or disease treatment purposes, and their purposes may also be for non-disease diagnosis, non-disease prognosis, and non-disease treatment purposes; their immediate purpose can be to obtain information on intermediate results of disease diagnosis result, disease prognosis result, and/or disease treatment result, and their immediate purpose can be non-disease diagnosis, non-disease prognosis, and/or non-disease treatment purposes.

CLL1, as described herein, a C-type lectin-like molecule 1, also known as C-type lectin domain family 12 member A (CLEC12A), is a type II transmembrane glycoprotein that plays an important role in immune regulation as an inhibitory receptor. CLL1 is present in myeloid cells of peripheral blood and bone marrow, as well as in most cells of acute myeloid leukemia (AML). It is also expressed on most $CD34^+CD38^-$ stem cells of AML, while normal $CD34^+CD38^-$ stem cells do not express it. Due to its unique expression pattern, CLL1 has become a potential target for AML treatment and diagnosis. CLL1 is also expressed on cells of myelodysplastic syndromes (MDS) and chronic myeloid leukemia (CML).

The present invention first uses recombinant CLL1 protein to immunize alpacas to obtain single-domain antibody gene, and constructs a single-domain antibody expression library, then screens and obtains anti-CLL1 single-domain antibodies (CLL1-VHH-16) with high affinity from the single-domain antibody expression library by phage display screening technology. After obtaining an antibody gene sequence by sequencing, CLL1-VHH-16 was prepared using genetic engineering methods. Further functional experiments were conducted on the affinity and specificity of the obtained antibodies. The results showed that the single-domain antibody of the present invention has high affinity and can highly specifically target CLL120 positive cells (cells expressing CLL1 protein), and can be successfully applied to detection of CLL1 expression in bone marrow cells of AML patients. The single-domain antibody of the present invention can be expressed and produced in prokaryotic cells, yeast cells, eukaryotic cells, and any recombinant system, and can be made into specific antibody drugs for prevention and treatment of CLL1 target related diseases (such as acute myeloid leukemia, myelodysplastic syndrome, or chronic myeloid leukemia) in clinical practice, or kits for detection of CLL1 protein. The single-domain antibody drug of the present invention has stable structure, small molecule size, easy recombinant expression, low production cost, and can be used alone or as a drug delivery system to carry related drugs. It has broad prospects and important significance in drug applications and clinical diagnosis.

**Brief Description of the Drawings**

Figure 1 shows the results of agarose gel electrophoresis of total RNA extracted from alpaca PBMC.

Figure 2 shows the results of agarose gel electrophoresis of PCR for bacterial library colony.

Figure 3 shows the results of CLL1 expression on different tumor cell lines detected using FITC labeled CLL1-VHH-16 antibody. Among them, Isotype represents CLL1-VHH-16-hFc antibody without FITC labeling, and CLL1 represents FITC-CLL1-VHH-16-hFc antibody.

Figure 4 shows the expression of CLL1 on bone marrow cells of AML patients detected using FITC labeled CLL1-VHH-16 antibody. In Figure 4, CLL1VHH-FITC represents FITC labeled CLL1-VHH-16 antibody, which is the FITC-CLL1-VHH-16-hFc antibody in Example 2; CLL1-APC represents APC labeled anti human CLL1 specific antibody from Biolegend (item number 143405).

**Detailed Description**

The present invention is further described in detail as below in conjunction with specific embodiments. The provided embodiments are only intended to illustrate the present invention and not to limit its scope. The Examples provided below can serve as a guide for those skilled in the art to further improve and do not in any way limit the present invention.

The experimental methods in the following examples, unless otherwise specified, are all conventional methods, carried out according to the techniques or conditions described in the literature in this field or according to the product manual. The materials, reagents, etc. used in the following examples can be obtained from commercial sources unless otherwise specified.

The pComb3XSS plasmid in the following examples is a product of Beijing Zoman Biotechnology Co., Ltd., catalog number ZK129.

The helper phage M13K07 in the following examples is a product of Chengdu Renyu Biotechnology Co., Ltd., catalog number A001-1.

The K562 cell (CLL1 negative cell) in the following examples is a product of Nanjing Cobioer Bioscience Co., Ltd., catalog number CBP60529; MM1. S cell (CLL1 negative cell) is a product of Beijing Biobw Biotechnology Co., Ltd., catalog number bio-129535; U937 cell (CLL1 positive cell) is a product of Beijing Biobw Biotechnology Co., Ltd., catalog number bio-73180.

The bone marrow cells of AML patients in the following examples were from Zhejiang Provincial People's Hospital.

The preparation method of CLL1 positive cell K562-CLL1 in the following example is to insert CLL1 between the *BamHI* and *EcoRI* sites of pcDNA3.1, and transfect the prepared recombinant vector into K562 cells to obtain CLL1 positive cell K562-CLL1.

The vector pcDNA3.1 in the following examples is a product of Changsha Abiowell Biotechnology Co., Ltd., catalog number: HG-VPI0001.

**Example 1: Preparation of anti-CLL1 single-domain antibody**

In this example, recombinant CLL1 protein was used to immunize alpacas to obtain a single-domain antibody gene, and a single-domain antibody expression library was constructed, and then, an anti-CLL1 single-domain antibody with high affinity was screened and obtained from single-domain antibody expression library by phage display screening technology.

1. Animal immunization

1.1 Acquisition of antigens

The recombinant CLL1 protein as an antigen is as below:

CLL1 immunization antigen: CLL1-hIgG1Fc (B918601) protein is a product of Shanghai Biointron Biotechnology Co., Ltd.

CLL1 screening antigen: CLL1-6×His (B918602) protein is a product of Shanghai Biointron Biotechnology Co., Ltd.

1.2. Immunization of Alpaca

Alpacas (2-3 years old, weighing around 50 Kg) were immunized with CLL1 immunization antigen CLL1-hIgG1Fc for 4 times, subcutaneously injecting 0.5 mg of CLL1-hIgG1Fc each time, with a 2-week interval between the two immunizations. Before the next immunization, 5 ml of blood was drawn, and the plasma was separated, followed by detection of the antibody titer after the previous immunization. The fourth immunizing valence after four immunizations is detected one week after injection.

The titer of antibodies in serum is detected by indirect ELISA method, the steps are as follows:

(1) the immunizing antigen CLL1-hIgG1Fc was diluted with PBS to 2 ug/ml, coated on an enzyme-linked immunosorbent assay plate at 100 $\mu$L/well, and incubated overnight at 4 °C; and the control group was coated with 3% BSA;

(2) the antigen solution was discarded and the enzyme-linked immunosorbent assay plate was rinsed with 200 $\mu$L PBST solution (1 x PBS containing 0.05% Tween20) for three times, each time for 5 minutes;

(3) the enzyme-linked immunosorbent assay plate was blocked with 200 $\mu$L of blocking solution PBSTB (PBST containing 2% BSA) at room temperature for 1.5 hours;

(4) the blocking solution was discarded and the enzyme-linked immunosorbent assay plate was rinsed with 200 $\mu$L PBST solution for three times, each time for 5 minutes;

(5) the serum to be tested was diluted in a gradient of 1:10, 1:100, 1:1000, 1:10000, 1:100000, 1:1000000, and 1:10000000 using a blocking solution, added to the enzyme-linked immunosorbent assay plate, 100 $\mu$L/well, and incubated at room temperature for 2 hours;

(6) the serum was discarded and the enzyme-linked immunosorbent assay (ELISA) plate was rinsed with 200 $\mu$L PBST solution for 4 times, each time for 5 minutes;

(7) goat anti llama antibody (goat anti-Llama IgG H&L (HRP), Abcam, ab112786) diluted with 1:10000 blocking solution was added to each well, 100 $\mu$L/well, and incubated at room temperature for 1 hour

(8) the detection antibody was discarded and the enzyme-linked immunosorbent assay (ELISA) plate was rinsed with 200 $\mu$L PBST solution for 4 times, each time for 5 minutes;

(9) 100 $\mu$L of TMB substrate solution was added to each well and let it develop for 2-3 minutes;

(10) 100 $\mu$L of reaction termination solution was added to each well;

(11) the absorbance at 450 nm was measured using a micorplate reader within 30 minutes. Positive well determination criteria: $OD_{450}$ reading of an immunization serum sample is greater than the $OD_{450}$ reading of a serum sample without immunization, and the reading is greater than 0.5.

1.3 Immunization Results

The test results of serum titer are shown in Table 1, in which bold font indicates positive wells. After four immunizations, the serum titer in the alpaca reached $10^5$, indicating an antibody level of $10^5$.

Table 1. Serum Titer Test Results

| Different antibody dilution | Ag Coating | | | | |
|---|---|---|---|---|---|
| | No immunization | First immunization | Second immunization | Third immunization | Fourth Immunization |
| 1:10 | 0.1497 | **1.5622** | **1.4494** | **1.5722** | **1.3833** |
| 1:100 | 0.0371 | **1.2325** | **2.0392** | **2.0212** | **1.9837** |
| 1:1000 | 0.0265 | 0.3245 | **2.0358** | **2.0520** | **2.1111** |
| 1:10000 | 0.0325 | 0.1251 | **1.6479** | **1.8318** | **1.8690** |
| 1:100000 | 0.0266 | 0.0911 | **0.7545** | **0.9391** | **0.9838** |
| 1:1000000 | 0.0205 | 0.0585 | 0.1141 | 0.1692 | 0.1929 |
| 1:10000000 | 0.0061 | 0.0172 | 0.0239 | 0.0882 | 0.0714 |
| PBSTB | 0.0000 | 0.0000 | 0.0000 | 0.0000 | 0.0000 |

As shown in Table 1, after the fourth immunization, the antibody level in the alpaca reached $10^5$. The Ag coating is a coating with the immunization antigen CLL1-hIgG1Fc. PBSTB is a blocking liquid.

2. Construction of phage library

After successful immunization, peripheral blood mononuclear cells (PBMCs) from alpaca were collected to construct a phage library, namely anti-CLL1 VHH phage antibody library.

2.1 RNA Extraction and Reverse Transcription

Peripheral blood mononuclear cells (abbreviation as alpaca PBMCs) of alpaca were isolated using a density gradient centrifugation method, dissolved and stored in Trizol. Total RNA was extracted from alpaca PBMC cells and the obtained total RNA was reverse transcribed into cDNA using Takara reverse transcription kit. The specific steps are as follows:

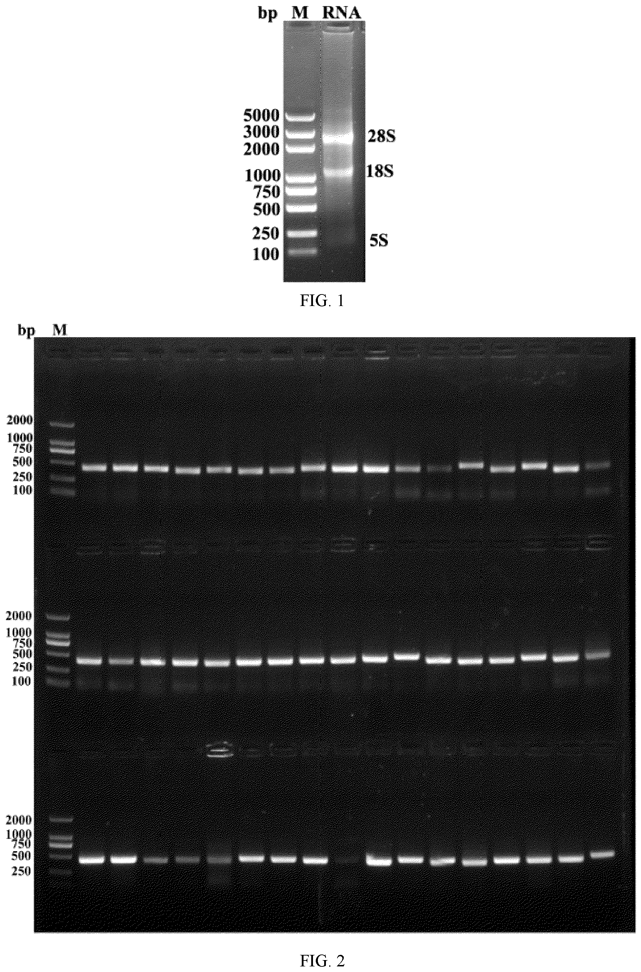

(1) transfer the alpaca PBMC cells stored in Trizol to a 1.5 mL centrifuge tube, add 1/5 volume of chloroform and mix well, let it stand at room temperature for 5 minutes;
(2) centrifuge at 4°C and 12000 g for 15 minutes;
(3) transfer the supernatant to a new centrifuge tube, add an equal volume of isopropanol, and let it stand at room temperature for 10 minutes;
(4) centrifuge at 4°C and 12000 g for 10 minutes;
(5) wash the precipitate with 1 mL of 75% ethanol, centrifuge at 7500 g for 5 minutes to remove ethanol, and dry;
(6) dissolve the precipitate in an appropriate amount of RNase free water to obtain extracted total RNA, obtaining a total of 181.2 ug of total RNA (Figure 1);
(7) reverse transcribe the total RNA using Takara reverse transcription kit: the total RNA sample was divided into two parts, one using Oligo dT Primer in the kit as a primer, and the other using Random 6-mers in the kit as a primer, follow the instructions in the kit to reverse transcribe the total RNA into cDNA, obtaining a total of 31.7 $\mu$g cDNA.

2.2 PCR of VHH target fragment of CLL1 antibody

Using 8 ng cDNA as a template, the VHH target fragment was amplified by conventional PCR, and a total of 83 $\mu$g was obtained.

2.3 Enzyme digestion and ligation

1. using pComb3XSS as a vector of phage plasmid, the pComb3XSS vector and PCR products of VHH (i.e. VHH target fragment) were digested with restriction endonucleases Spe I and Sac I, respectively, and incubated at 37°C for 4 hours;
2. the pComb3XS S vector the PCR products of VHH after enzyme digestion using a DNA recovery purification kit were purified, and store at 4 °C.
3. the pComb3XSS vector and the VHH target fragment after double enzyme digestion were ligated and recovered, obtaining a total of 3.1 $\mu$g of the ligated product.

2.4. Construction of Bacterial Library

(1) Take 100 ng of the ligated product together with 50 $\mu$L of TG1 competent cells for electroporation;
(2) recover at 37 °C for 1 hour, take 100 $\mu$L of the transformed product and coat the plate after dilution of 10 gradients, and incubate overnight at 37 °C;
(3) calculate the number of transformed colonies obtained from all electroporation reactions based on dilution factor and single colony count, that is, the library capacity of the bacterial library is $2.6 \times 10^8$ cfu;
(4) randomly select several single clones from the gradient dilution plate for colony PCR, and the agarose gel electrophoresis results showed (Figure 2) that the selected singe clones were all positive clones, with a positive rate of 100%;
(5) collect all remaining bacterial solutions (i.e. gradient dilutions of transformation products) and evenly spread them onto 20 culture plates of 15 cm (2×YT medium, containing 100 $\mu$g/mL Amp and 2% agarose), invert and culture overnight at 37 °C;
(6) the overnight cultured plate colonies were scraped off with $2 \times$ YT liquid culture medium and placed in a 50 mL

centrifuge tube, with $OD_{600}$ measured to be 120, and stored at -80 °C after the final concentration of 20% glycerol was added, the bacterial library was obtained.

(7) The sequencing sequences of the above monoclonal positive colonies were translated into protein sequences, and the sequence diversity alignment showed that they were all independent sequences with good diversity. The bacterial library diversity meets the requirements.

### 2.5. Preparation of phage library

(1) Inoculate the bacterial library into 100 mL of 2×YT liquid medium (containing 100 μg/mL Amp), culture at 37°C and 250 rpm until the $OD_{600}$ is 0.5-0.55;

(2) add helper phage M13K07 in a ratio of 1:20 bacteria to phages, and incubate at 37°C and 250 rpm for 30 minutes;

(3) add Kan at a final concentration of 50 μg/mL and incubate overnight at 30 °C and 250 rpm;

(4) centrifuge the overnight cultured bacterial solution at 4°C and 4000 rpm for 20 minutes, and transfer the supernatant to a new 50 mL centrifuge tube;

(5) add 1/4 volume of 20% PEG/2.5M NaCl solution pre-cooled at 4°C, mix well, and incubate on ice for at least 30 minutes;

(6) centrifuge at 4°C and 4000 rpm for 20 minutes, discard the supernatant, and invert for 2 minutes;

(7) add 1 mL of PBS to resuspend the pellet, centrifuge at 4 °C and 12000 rpm for 10 minutes;

(8) transfer the supernatant to a new centrifuge tube, add 1/4 volume of 20% PEG/2.5M NaCl solution pre-cooled at 4 °C, mix well to form a white suspension, and incubate on ice for at least 10 minutes;

(9) centrifuge at 4°C and 12000 rpm for 10 minutes, discard the supernatant, resuspend in 1 mL PBS, centrifuge at 12000 rpm for 10 minutes, transfer the supernatant to a new centrifuge tube, and store at -80°C to obtain the purified phage library with a titre of $1 \times 10^{13}$ pfu/mL.

### 3. Affinity panning

3 rounds of affinity screening on the phage library were performed using the screening antigen (CLL1-6 × His), including titer detection, amplification of phage eluate, and purification of phage.

### 3.1. First round of screening

### 3.1.1. First round screening method

(1) Coat an immune tube (50 μg/tube, coated with 2 mL/tube of PBS) with a screening antigen (CLL1-6×His), slowly rotate overnight at 4 ° C, and parallel coat with BSA (50 μ g/tube, coated with 2 mL/tube of PBS) as a control;

(2) discard the liquid from the overnight coated immune tube, add 2 mL of PBS buffer and wash 3 times at room temperature, rotating for 5 minutes each time;

(3) add 2 mL of 3% skim milk powder and block with rotation at room temperature for 2 hours;

(4) discard the liquid from the immune tube after blocking, add 2 mL of PBST buffer and wash 3 times at room temperature, rotating for 5 minutes each time;

(5) discard the washing solution from the immune tube and add the phage library as input phage library for the first round of screening according to the following formula, add PBS buffer to 2 mL and incubate at room temperature for 1 hour.

$$V(ul) = \frac{10^{12}}{T_{library}} \times 1000$$

Wherein, V is volume of the added phages (in μL), and $T_{library}$ is phage titre.

(6) discard the liquid inside the immune tube and add 2 mL of PBST (1×PBS plus 0.1% Tween20, the same below) buffer, wash the immune tube 20 times at room temperature, rotating for 5 minutes each time;

(7) discard the liquid inside the immune tube, add 1 mL of 0.25 mg/mL Trypsin solution, and spin for elution at room temperature for 30 minutes;

(8) add 10 μ L of 10% AEBSF (serine protease inhibitor) to terminate the elution, and transfer the solution in the immune tube to a new 1.5 mL centrifuge tube, which is the phage eluate for the first round of screening (referred to as the first round phage eluate).

3.1.2. Initial titer detection of phages

(1) TG1 strain stored in a -80 °C refrigerator was streaked with single colonies on 2×YT solid medium and incubated overnight at 37°C;
(2) pick one single bacterium clony from a single colony plate and place it in 5 mL of 2×YT medium, incubate overnight at 37°C;
(3) transfer 50 μL of bacterial solution to 5 mL of 2×YT liquid culture medium, culture at 37°C and 250 rpm until $OD_{600}$=0.5-0.55, obtaining TG1 bacterial solution;
(4) take 10 μL of the first round of phage eluate and dilute it in a 1.5 mL centrifuge tube at a 10 fold gradient, that is, take 10 μL of the first round of phage eluate and add it to 90 μL of PBS, from which then take 10 μL and add to 90 μL of PBS, and so on, a total of 12 gradients, dilute to $10^{-12}$;
(5) add 90 μL of TG1 bacterial solution to each dilution centrifuge tube, shake and mix well, and incubate at 37°C for 30 minutes;
(6) take 5 μL of bacterial liquid droplets from each dilution centrifuge tube, add them to 2×YT solid culture medium (Amp), stand for a few minutes, invert at 37°C overnight;
(7) count the number of clearly distinguishable single colonies in the plate at different dilutions, and calculate the amount of phages per milliliter of phage solution according to the following formula, which is the titre of the phage library:

$$T \ (pfu/ml) \ = N \ \times D \ \times 400$$

wherein, T is phage titre (in pfu/mL), D is dilution factor, and N is the number of single colonies in the plate of corresponding dilution factor.

3.1.3. Amplification of the first round of phage eluate

(1) TG1 strain stored in a -80°C refrigerator was streaked with single colonies on 2×YT solid medium and incubated overnight at 37°C;
(2) pick one single bacterium clony from a single colony plate and place it in 5 mL of 2×YT medium, incubate overnight at 37°C;
(3) transfer 50 μL of bacterial solution to 5 mL of 2×YT liquid culture medium, culture at 37°C and 250 rpm until $OD_{600}$=0.5-0.55;
(4) add 500 μL of the first round phage eluate and continue to culture at 37 °C and 250 rpm for 30 minutes;
(5) spread all bacterial liquid evenly onto three 15cm circular culture medium plates containing 100 μg/mL Amp, and incubate overnight at 37°C;
(6) add 2×YT liquid culture medium to the surface of the culture medium plate, gently scrape off the colonies with a coating rod, and collect the bacterial liquid into a 15 mL centrifuge tube, i.e. an amplified bacterial sublibrary, add glycerol with a final concentration of 20%, and measure the $OD_{600}$ value of the bacterial liquid using a spectrophotometer to obtain the $OD_{600}$ value of a bacterial library of eluate;
(7) transfer the bacterial library of eluate to 100 mL of 2×YT liquid medium (containing 100 μg/mL Amp) according to the following formula, with an initial $OD_{600}$ of 0.1:

$$V \ (ul) = \frac{10}{OD600} \times 1000$$

wherein, V is the volume of the transferred bacterial liquid (unit: μL), and $OD_{600}$ is the $OD_{600}$ of the constructed bacterial library of eluate;
(8) culture at 37°C and 250 rpm until the $OD_{600}$ of the bacterial liquid is 0.5-0.55;
(9) add helper phage M13K07 according to the following formula with a ratio of bacteria: bacteriophages=1:20:

$$V\ (ml)\ =\ \frac{OD600\ \times 1.6\ \times 10^{11}}{T_{helper-phage}}$$

wherein, V is volume of the helper phage added (in mL), $T_{helper-phage}$ is titer of the helper phage, and $OD_{600}$ is the $OD_{600}$ value of the bacterial liquid;

(10) continue to cultue at 37°C and 250 rpm for 30 minutes;

(11) add Kan with a final concentration of 50 µg/mL and incubate at 30°C and 200 rpm overnight.

3.1.4. First round of phage purification

(1) transfer the overnight cultured bacterial solution to a new 50 mL centrifuge tube and centrifuge at 4°C and 4000 rpm for 20 minutes;

(2) transfer the supernatant to a new 50 mL centrifuge tube, add 1/4 volume of 20% PEG/2.5M NaCl solution pre-cooled at 4 °C, mix thoroughly, and place it on ice for at least 30 minutes;

(3) centrifuge at 4 °C and 4000 rpm for 20 minutes, discard the supernatant;

(4) add 1 mL of PBS to resuspend the precipitate and transfer it to a new 1.5 mL centrifuge tube, and centrifuge at 4 °C and 13000 rpm for 15 minutes;

(5) transfer the supernatant to a new 1.5 mL centrifuge tube, add 1/4 volume of 20% PEG/2.5M NaCl solution pre-cooled at 4 °C, mix well, and place it on ice for at least 10 minutes;

(6) centrifuge at 4 °C and 13000 rpm for 10 minutes, discard the supernatant, and add 1 mL of PBS to resuspend the precipitate;

(7) centrifuge at 4 °C and 13000 rpm for 2 minutes, transfer the supernatant to a new 1.5 mL centrifuge tube, which is the first round screening phage sublibrary. It should be stored at -80 °C for a long time and at -20 °C for a short period of time (1-2 weeks).

3.1.5. Titer detection of first round screening phage sublibraries

The method is the same as step 3.1.2 for initial titer detection of phages.

3.2. Second round of screening

3.2.1. Second round screening method

The screening method is the same as the first round screening method in step 3.1.1. The phage sublibrary obtained by amplification and purification in the first round of screening (first round screening phage sublibrary) is used as an input phage library for the second round of screening, and the phage eluate for the second round of screening is obtained.

3.2.2. Titer detection of the second round of phage eluate

The detection method is the same as step 3.1.2 for phage initial titer detection.

3.2.3 Amplification and purification of second round phage eluate

Method is the same as amplification and purification of the first round of phage eluate in steps 3.1.3 and 3.1.4, and the second round screening phage sublibrary obtained.

3.2.4. Titer detection of the second round screening phage sublibrary

The method is the same as step 3.1.5 for titer detection of the first round screening phage sub libraries.

3.3 Third round of screening

3.3.1 Third round screening method

The screening method is the same as the first round screening method in step 3.1.1. In the second round of screening, the phage sublibrary obtained by amplification and purification (second round screening phage sublibrary) is used as an input phage library for the third round of screening, and the phage eluate for the third round of screening is obtained.

3.3.2. Titer testing of the third round of phage eluate

The detection method is the same as step 3.1.2 phage initial titer detection.

3.3.3 Amplification of the third round eluate

The method is the same as amplification of the first round of phage eluate in step 3.1.3, and the third round of amplified phage eluate is obtained.

3.4 Three rounds of screening results

The results of the three rounds of affinity screening are shown in Table 2:

Table 2: Three rounds of affinity screening results

| Rounds of Screening | Input titer | Screening titer | Control titer | Enrichment degree*1 | Difference multiple*2 |
|---|---|---|---|---|---|
| First Round | $1 \times 10^{12}$ pfu | $2 \times 10^7$ pfu | $2 \times 10^6$ pfu | $2 \times 10^{-5}$ | 10 |
| Second Round | $6.6 \times 10^{12}$ pfu | $3.6 \times 10^{10}$ pfu | $2 \times 10^6$ pfu | $5.5 \times 10^{-3}$ | 18000 |
| Third Round | $4.0 \times 10^{12}$ pfu | $5.2 \times 10^{10}$ pfu | $6.0 \times 10^6$ pfu | $1.3 \times 10^{-2}$ | 8600 |

Wherein, the enrichment degree of * 1 is the screening titer divided by the input titer, and the larger the value, the higher the enrichment degree of the antibody in the input library; *the difference multiple of 2 is the screening titer divided by the control titer, the higher the value, the higher the content of positive antibodies in the screening library.

The results showed that after 3 rounds of screening, the enrichment degree reached $1.3 \times 10^{-2}$ times, and ELISA monoclonal validation could be performed.

4. Selection of CLL1 VHH positive monoclonal antibodies

4.1 ELISA detection of CLL1 VHH positive monoclones

(1) Take a suitable dilution of bacterial fluid from the third round of amplified phage eluate and evenly spread it onto a solid culture medium plate containing 100 μ g/mL Amp, incubate overnight at 37°C;
(2) randomly pick 192 monoclonal colonies into 96 well cell culture plates (P1-P2), add 200 μL of 2×YT medium (containing 100 μg/mL Amp) to each well, and culture overnight at 37°C;
(3) transfer 5 μL of overnight cultured bacterial liquid to a new 96 well cell culture plate with 200 μL of 2×YT liquid medium (containing 100 μg/mL Amp) added to each well, and incubate at 37 °C for 5 hours;
(4) add helper phage M13K07 according to the following formula in a ratio of number of bacteria: number of phages=1:20:

$$V\ (ml) = \frac{\text{OD600}\ \times 1.6\ \times 10^{11}}{T_{helper-phage}}$$

wherein, V is volume (in mL) of the helper phage added, and $T_{helper\ phage}$ is titer of the helper phage;

(5) incubate at 37°C for 30 minutes, add Kan at a final concentration of 50 μg/mL, and incubate overnight at 30°C;

(6) centrifuge the overnight cultured bacterial liquid at 4°C and 4000 rpm for 10 minutes to obtain a supernatant, and store it at 4°C for future use;

(7) dilute the screening antigen with PBS to 1 ug/ml, coat it on an enzyme-linked immunosorbent assay plate, 100 μL/well, and incubate overnight at 4°C; control group was coated with 3% BSA;

(8) discard the antigen solution and add 200 μL of PBS buffer to each well to wash the enzyme-linked immunosorbent assay plate at room temperature for 3 times, 10 minutes for each time;

(9) add 200 μL of blocking solution PBSTB (PBST containing 2% BSA) to each well to block the enzyme-linked immunosorbent assay plate, and block at room temperature for 1 hour;

(10) discard the blocking solution and add 200 μL of PBST (1×PBS containing 0.1% Tween20) buffer to each well, wash the enzyme-linked immunosorbent assay plate for 3 times at room temperature, 10 minutes for each time;

(11) add 100 μL of blocking solution to each well, followed by 100 μL of the supernatant obtained from centrifugation in step (6), and incubate at room temperature for 2 hours;

(12) discard the liquid in the enzyme-linked immunosorbent assay plate and wash each well with 200 μL of PBST buffer for 3 times, each time for 10 minutes;

(13) add M13 Bacteriophage HRP Antibody, Mouse Mab (product of Sino Biological Inc., catalog number 11973-MM05T-H) diluted in blocking solution at a ratio of 1:30000 to each well, 100 μL/well, and incubate at room temperature for 1 hour;

(14) discard the liquid in the ELISA plate, add 200 μL PBST buffer to each well and wash 6 times, each time for 5 minutes;

(15) add 100 μL of TMB single component colorimetric solution to each well, and incubate in the dark for 1-3 minutes. Add 100 μL of 1 M HCl to each well to stop the reaction, and read the $OD_{450}$ value using a micorplate reader.

$OD_{450}>0.5$ is considered as a positive result, and the positive clones obtained are phages containing single-domain antibodies that can bind to CLL1 protein.

4.2. ELISA secondary validation of CLL1 VHH positive monoclones

To eliminate false positive results, the clones initially identified as positive were subjected to ELISA secondary validation, the method is same as step 4.1.

4.3 Sequencing of CLL1 VHH positive monoclones

Take 5 μL of bacterial solution of 181 positive clone from the ELISA secondary detection plate and inoculate it into 1 mL of 2×YT medium (containing 100 μg/mL Amp), respectively. Incubate at 37 °C and 250 rpm until $OD_{600}=0.8$-1.0. Take 0.5 mL of bacterial solution for sequencing and obtain different antibody sequences.

5. Preparation of anti-CLL1 single-domain antibody

5.1 Structure and sequence of anti-CLL1 single-domain antibody

Select the positive monoclones in step 4.3 and sequence them to obtain an antibody gene sequences, and utilize genetic engineering methods to prepare anti-CLL1 single-domain antibodies (CLL1-VHH -16), naming one of the antibodies as CLL1-VHH-16.

Single-domain antibodies only have the variable region (VHH) of heavy chain antibodies, which is composed of framework region FR1, complementarity determining region CDR1, framework region FR2, complementarity determining region CDR2, framework region FR3, complementarity determining region CDR3, and framework region FR4 in sequence. The specific sequence information is as follows:

The amino acid sequence of the single-domain antibody CLL1-VHH-16 is SEQ ID No.1. In SEQ ID No.1, the 1-25 positions are the frame region FR1, the 26-35 positions are the complementary determining region CDR1, the 36-49 positions are the frame region FR2, the 50-59 positions are the complementary determining region CDR2, the 60-98 positions are the frame region FR3, the 99-118 positions are the complementary determining region CDR3, and the 119-129 positions are the frame region FR4. The nucleotide sequence of the nucleic acid molecule encoding the single-domain antibody CLL1-VHH-16 (CLL1-VHH-16 gene) is shown in SEQ ID No.2.

The amino acid sequence of the single-domain antibody CLL1-VHH-16: 5'-DVQLQESGGGLVQPGGSLRLS CAAS<u>GFTFGSYDMT</u>WVRQAPGKGPEWVS<u>GINSGGSST</u> YYADSVKGRFTISRDNAKNTLYLQMNSLKPEDTAVYYCA TIDR<u>LSTVVAGTYQPSEEYD</u> YWGQGTQVTVSS-3'(SEQ ID No.1). The three underlined parts are CDR1, CDR2, and CDR3 in sequence.

The nucleotide sequence of CLL1-VHH-16 gene: 5'-GATGTGCAGCTGCAGGAGTCTGGAGGAGGCTTGGT GCAGCCTGGGGGTTCTCTGAG ACTCTCCTGTGCAGCCTCTGGATTCACCTTCGGAAGCTATGACATGACCTGGG TCCGC CAGGCTCCAGGAAAGGGGCCCGAGTGGGTCTCAGGAATTAATAGTGGTGGTAGTAGC ACATACTATGCA GACTCCGTGAAGGGCCGATTCACCATCTCCAGAGACAACGCCAAG AACACGCTGTATCTGCAAATGAACAGCCT GAAACCTGAGGACACGGCCGTGTATTAC TGTGCAACTATCGACCGCCTTAGTACGGTAGTAGCTGGTACGTACCA GCCGTCCGAAG AATATGACTACTGGGGCCAGGGGACCCAGGTCACCGTCTCCAGC-3'(SEQ ID No.2).

The sequence of complementary determining regions is defined according to the Kabat numbering system.

The above-mentioned single-domain antibody is a single-domain antibody that specifically binds to CLL1 protein.

5.2 Expression and purification of anti-CLL1 single-domain antibody

An eukaryotic expression vector pcDNA3.1 was used to express the anti-CLL1 single-domain antibody (CLL1-VHH-16).

5.2.1 Construction of a recombinant vector

In order to facilitate purification, a human derived IgG1Fc gene (hFc gene, nucleotide sequence thereof is SEQ ID No. 3, encoding amino acid sequence thereof is SEQ ID No. 4) was added to the 3'end of the coding gene (SEQ ID No.2) of the single-domain antibody CLL1-VH-16 to obtain a CLL1-VHH16-hFc fusion gene. Then, a *BamHI* enzyme cleavage site was added before the 5' start codon and an *EcoRI* enzyme cleavage site was added after the 3'end stop codon of the fusion gene. A recombinant eukaryotic expression vector pcDNA3.1- CLL1-VHH-16-hFc expressing the single-domain antibody CLL1-VHH-16 fused with hFc gene at C-terminal was obtained by cloning the resulting fusion gene into as site between *BamHI* and *EcoRI* recognition sites of an eukaryotic expression vector pcDNA3.1.

5.2.2 Expression of anti-CLL1 single-domain antibody

The eukaryotic expression vector pcDNA3.1-CLL1-VHH-16-hFc obtained in step 5.2.1 was transfected into HEK293 cells using conventional electroporation methods for expression.

5.2.3 Purification of anti-CLL1 single-domain antibody

Protein A has the activity of binding to Fc fragments of IgG, therefore, Protein A column was used to purify the anti-CLL1 single-domain antibody expressed in HEK293 cells (conventional purification steps).

Finally, purified anti-CLL1 single-domain antibody fused with hFc gene (CLL1-VHH-16-hFc) was prepared.

**Example 2: Functional identification of anti-CLL1 single-domain antibody**

1. Determination of antigen-antibody affinity constants

Antibody to be tested: the anti-CLL1 single-domain antibody (CLL1-VHH-16-hFc) fused with hFc gene prepared in step 5.2.3.

(1) The antibody to be tested CLL1-VHH-16-hFc is labeled with Pierce™ NHS Fluorescence Antibody Labeling Kit to obtain a FITC direct labeled antibody FITC-CLL1-VHH-16-hFc for flow cytometry detection.

(2) Resuspend CLL1 positive cells K562-CLL1 and U937 cells to a density of $2\times10^6$ cells/mL, take 100 $\mu$L and place them in a 96 well V-plate, respectively, centrifuge and discard the supernatant, and collect the cells (with K562 cells as the CLL1 negative cell control).

(3) Mix 40 $\mu$L of FITC-CLL1-VHH-16-hFc diluted in different gradients (specific grouping shown in Table 3) with the above cells and incubate at room temperature in the dark for 10 minutes.

Table 3: Grouping of CLL1-VHH-16-hFc Antibody Affinity Test

| | Antibody concentration ($\mu$g/mL) | | | | | | | |
|---|---|---|---|---|---|---|---|---|
| K562-CLL1 | 5.7 | 2.85 | 1.425 | 0.712 5 | 0.3563 | 0.178 1 | 0.0891 | 0.0445 |
| Antibody dilution ratio | 1:100 | 1:200 | 1:400 | 1:800 | 1:1600 | 1:320 0 | 1:6400 | 1:12800 |

(4) Wash twice with 200 $\mu$L FACS buffer by centrifugation, resuspend in 200 $\mu$L FACS buffer, and immediately perform flow cytometry to measure the mean fluorescence intensity (MFI).

(5) Calculate the equilibrium dissociation constant (KD) of CLL1 VHH antibody binding to target cells according to the formula to determine the strength of affinity. Wherein, the smaller the KD value, the stronger the affinity.

$$1/(MFI-Con)=1/Fmax+(KD/Fmax) (1/[CLL1\text{-}scfv\ IgG\ Fc]).$$

Wherein, MFI-Con represents the relative average fluorescence intensity, which is the value obtained by subtracting the background average fluorescence intensity from the average fluorescence intensity of the experimental group; CLL1-scfv IgG Fc represents the amount of antibody used (unit: ng).

A standard curve is drawn with the reciprocal of antibody usage as x-axis and the reciprocal of relative average fluorescence intensity as y-axis to obtain a linear regression equation. According to the linear regression equation, the intersection point between the standard curve and the vertical axis is 1/Fmax, and KD is the slope of the line multiplied by Fmax.

The calculated equilibrium dissociation constants are shown in Table 4:

Table 4: Equilibrium dissociation constants (KD) of CLL1-VHH-16-hFc and K562-CLL1 cells

| single-domain antibody | Affinity constant KD (nM) | Linear regression equation | Correlation coefficient of linear regression $R^2$ |
|---|---|---|---|
| CLL1-VHH-16 | 5 | y=0.004x+0.0004 | 0.999 |

2. Specificity detection of single-domain antibody CLL1-VHH-16

Expression of CLL1 on different tumor cells were detected using FITC labeled CLL1-VHH-16 antibody (FITC-CLL1-VHH-16-hFc).

(1) CLL1 negative cell lines (control group): K562 cells and MM1. S cells; CLL1 positive cell lines (experimental group): U937 cells and CLL1 overexpressing cell line K562-CLL1, were all revived at 37°C and added to cell culture flasks. They were cultured at 37°C and 5% $CO_2$ for 2 passages before being used to detect anti-CLL1 single-domain antibodies (FITC-CLL1-VHH-16-hFc).

(2) Adjust the concentration of each cell to $2 \times 10^6$ cells/mL, add 200 $\mu$L/well to a 96 well plate, centrifuge at 1500 rpm for 5 minutes.

(3) Wash with FACS buffer and centrifuge.

(4) Add 20 $\mu$L diluted FITC-CLL1-VHH-16-hFc antibody (1:400) and mix well. Incubate at room temperature for 10 minutes.

(5) Add FACS buffer and centrifuge to wash away antibodies, then resuspend in buffer and detect using flow cytometry.

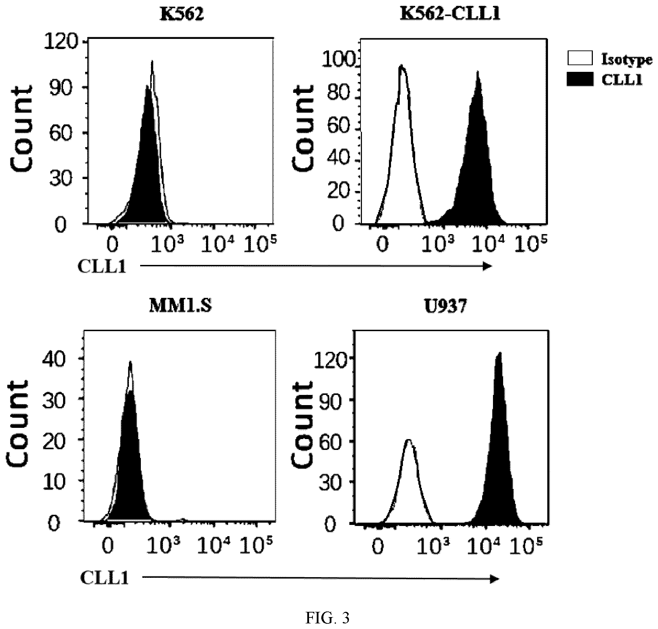

The flow cytometry results showed that FITC-CLL1-VHH-16-hFc antibody can specifically bind to the CLL1 antigen molecule expressed on tumor cells (Figure 3).

3. Detection of CLL1 expression in bone marrow cells of AML patients

FITC labeled CLL1-VHH-16 antibody (FITC-CLL1-VHH-16-hFc) was used to detect bone marrow cells from three patients with acute myeloid leukemia (AML), and compare them with the specific APC labeled anti-human CLL1 antibody (catalog number 143405) from Biolegend. The flow cytometry analysis steps are as follows:

(1) Revive the frozen bone marrow cells of three AML patients at 37°C.

(2) Adjust the cell density to $2\times10^6$ cells/ml, add 200 $\mu$ L/well to a 96 well plate, and centrifuge at 1500 rpm for 5 minutes.

(3) Wash with FACS buffer and centrifuge.

(4) Add 20 $\mu$ L diluted FITC-CLL1-VHH-16-hFc antibody (1:400) and mix well. Incubate at room temperature for 10 minutes.

(5) Add FACS buffer and centrifuge to wash away antibodies, then resuspend in buffer and detect using flow cytometry.

(6) APC labeled anti-human CLL1 specific antibody (CLL1-APC) of Biolegend was used as a control, following the same steps as above.

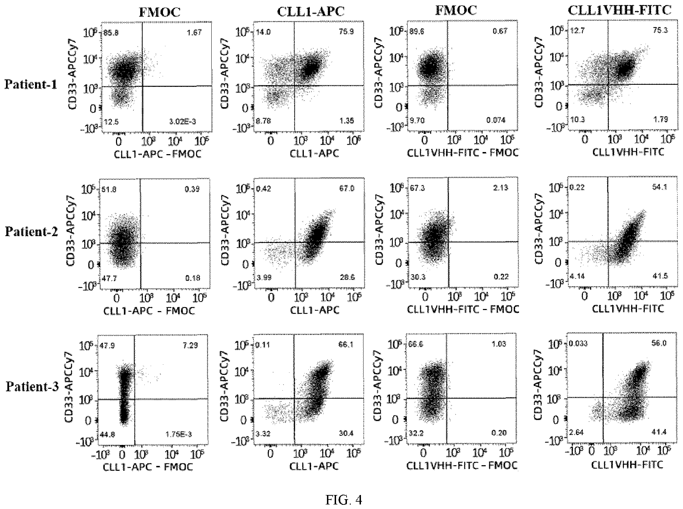

The results showed that both the FITC-CLL1-VHH-16-hFc antibody of the present invention and the APC labeled anti-human CLL1 specific antibody of Biolegend had similar expression profiles (Figure 4), indicating that the single-domain antibody CLL1-VHH-16 of the present invention can specifically recognize CLL1 expressed on AML tumor cells.

The present invention has been described in detail above. For those skilled in the art, the present invention can be implemented within a wide range under equivalent parameters, concentrations, and conditions without departing from the purpose and scope of the present invention, and without the need for unnecessary experiments. Although the present invention provides specific examples, it should be understood that further improvements can be made to the present invention. In summary, according to the principles of the present invention, the present application is intended to include any modifications, uses, or improvements to the present invention, including changes made using conventional techniques known in the art that depart from the scope already disclosed in this application. According to the scope of the attached claims, some basic features can be applied.

**Industrial applications**

The anti-CLL1 single-domain antibody (CLL1-VHH-16) of the present invention has high affinity and can specifically target to CLL1 positive cells (cells expressing CLL1 protein), and can be successfully applied to detect the expression of CLL1 in bone marrow cells of AML patients. The single-domain antibody of the present invention can be prepared to specific antibody drugs clinically used for the prevention and treatment of CLL1 target related diseases, such as acute myeloid leukemia, myelodysplastic syndrome, or chronic myeloid leukemia. It can also be used to prepare CAR cells targeting CLL1 or to prepare detection kits for CLL1 protein. The single-domain antibody drug of the present invention has stable structure, small molecule size, easy recombinant expression, low production cost, and can be used alone or as a drug delivery system to carry related drugs. It has broad prospects and important significance in drug applications and clinical diagnosis.

**Claims**

1. A single-domain antibody, **characterized in that** the single-domain antibody is composed of a heavy chain variable region, the heavy chain variable region comprises complementary determining region CDR1 having an amino acid sequence of positions 26-35 of SEQ ID No.1, complementary determining region CDR2 having an amino acid sequence of positions 50-59 of SEQ ID No.1, and complementary determining region CDR3 having an amino acid sequence of positions 99-118 of SEQ ID No.1.

2. The single-domain antibody according to claim 1, **characterized in that** the amino acid sequence of the heavy chain variable region is SEQ ID No.1, or an amino acid sequence having at least 80% identity with SEQ ID No.1 and having the same function.

3. A biomaterial related to the single-domain antibody according claim 1 or 2, **characterized in that** the biomaterial is any one of the following C1) to C4):

   C1) a nucleic acid molecule encoding the heavy chain variable region of the single-domain antibody as claimed in claim 1 or 2;
   C2) an expression cassette containing the nucleic acid molecule described in C1);
   C3) a recombinant vector containing the nucleic acid molecule described in C1), or a recombinant vector containing the expression cassette described in C2);
   C4) a recombinant host cell containing the nucleic acid molecule described in C1), or a recombinant host cell containing the expression cassette described in C2), or a recombinant host cell containing the recombinant vector described in C3).

4. The biomaterial according to claim 3, **characterized in that** the nucleic acid molecule is any one of the following:

   D1) a DNA molecule having a nucleotide sequence or coding sequence of SEQ ID No.2;
   D2) a DNA molecule that has 75% or more identity with the nucleotide sequence defined by D1) and has the same function.

5. A pharmaceutical composition comprising the single-domain antibody according to claim 1 or 2.

6. An agent or a kit, **characterized in that** the agent or kit contains the single-domain antibody according to claim 1 or 2, the agent or kit has any one of the following uses:

F1) detection of CLL1 protein;
F2) screening, diagnosis, or auxiliary diagnosis of CLL1 target related diseases;
F3) use for in vivo imaging of CLL1 protein.

7. Any of the following uses of the single-domain antibody according to claim 1 or 2 and/or the biomaterial according to claim 3 or 4:

E1) use in the preparation of a medicament for prevention or treatment of a tumor, or in the prevention or treatment of a tumor;
E2) use in the preparation of a medicament for prevention or treatment of a CLL1 target related disease, or in the prevention or treatment of a CLL1 target related disease;
E3) use in the preparation of a product for screening, diagnosing, or assisting in the diagnosis of CLL1 target related diseases, or in the screening, diagnosis, or assisting in the diagnosis of CLL1 target related diseases;
E4) use in detecting a CLL1 protein or preparing a product for detecting a CLL1 protein;
E5) use in the preparation of a product for binding to CLL1 protein;
E6) use in mediating drug specific recognition of a tumor expressing a CLL1 antigen or in preparing a product that mediates drug specific recognition of a tumor expressing a CLL1 antigen;
E7) use in *in vivo* imaging of a CLL1 protein or preparation of a product for in vivo imaging of a CLL1 protein;
E8) use in the preparation of CAR cells targeting CLL1.

8. The use according to claim 7, **characterized in that** the CLL1 target related disease is a CLL1 positive cancer.

9. The use according to claim 8, **characterized in that** the CLL1 positive cancer is acute myeloid leukemia, myelodysplastic syndrome, or chronic myeloid leukemia.

10. A method for preparing the single-domain antibody according to claim 1 or 2, **characterized in that** the method comprises: constructing a recombinant expression vector containing the nucleic acid molecule according to claim 3 or 4; introducing the recombinant expression vector into host cells to obtain recombinant cells expressing the single-domain antibody; culturing the recombinant cells and obtaining the single-domain antibody through isolation and purification.

11. A method for diagnosing or assisting in the diagnosis of a CLL1 target related disease, **characterized in that** the method comprises: isolating a sample from a subject, then using the single-domain antibody according to claim 1 or 2 to detect the content of CLL1 protein in the sample, and diagnosing or assisting in the diagnosis of the CLL1 target related diseases based on the content of the CLL 1 protein.

12. A method for screening CLL1 target related diseases, **characterized in that** the method comprises: isolating a sample from a subject, then using the single-domain antibody according to claim 1 or 2 to detect the content of CLL1 protein in the sample, and screening for CLL1 target related diseases based on the content of the CLL1 protein.

13. A method for preventing or treating a CLL1 target related disease, **characterized in that** the method comprises administering the single-domain antibody of claim 1 or 2 or the pharmaceutical composition of claim 5 to a subject suffering from the CLL1 target related disease.

14. The method according to any one of claims 11-13, **characterized in that** the CLL1 target related disease is a CLL1 target related tumor.

15. The method according to claim 14, **characterized in that** the CLL1 target associated tumor is a CLL1 positive cancer.

16. The method according to claim 15, **characterized in that** the CLL1 positive cancer is acute myeloid leukemia, myelodysplastic syndrome, or chronic myeloid leukemia.

17. A method for detecting CLL1 protein in vitro, **characterized in that** the method comprises detecting CLL1 protein using the single-domain antibody according to claim 1 or 2 or the kit according to claim 6.

18. An antibody drug conjugate comprising an antibody portion and a conjugate portion, **characterized in that** the antibody portion comprises the single domain antibody according to claim 1 or 2.

19. The antibody drug conjugate according to claim 18, **characterized in that** the conjugate portion comprises a detectable label, a chemical drug, or a cytotoxic agent.

FIG. 1

FIG. 2

FIG. 3

FIG. 4

## INTERNATIONAL SEARCH REPORT

| International application No. |
| --- |
| **PCT/CN2022/143892** |

**A. CLASSIFICATION OF SUBJECT MATTER**

C07K16/28(2006.01)i; A61K47/68(2017.01)i; A61K49/00(2006.01)i; A61K51/10(2006.01)i; C12N15/13(2006.01)i; C12N15/85(2006.01)i; G01N33/574(2006.01)i; C12N5/10(2006.01)i

According to International Patent Classification (IPC) or to both national classification and IPC

**B. FIELDS SEARCHED**

Minimum documentation searched (classification system followed by classification symbols)

IPC:C07K A61K C12N G01N

Documentation searched other than minimum documentation to the extent that such documents are included in the fields searched

Electronic data base consulted during the international search (name of data base and, where practicable, search terms used)

CJFD, CNTXT, DWPI, ENTXT, ENTXTC, VEN, WPABS, WPABSC, CNKI, 万方数据库, WANFANG DATABASE, BAIDU 学术, BAIDU Scholar, PUBMED, ELSEVIER, SPRINGER, ISI WEB OF KNOWLEDGE, SCIENCEDIRECT, NCBI, Genbank, EMBL, STN, 智能化检索系统序列检索, intelligent search system sequence search: C型凝集素样分子1, C-type lectin-like molecule 1, CLL1, CLL-1, CLEC12A, C-type lectin domain family 12 member A, 单域抗体, 重链抗体, 纳米抗体, HCAB, VHH, SDAB, nanobody, CLL1-VHH-16, 浙江康佰裕生物科技有限公司, 发明人, inventor, GFTFGSYDMT, GINSGGSSTY, IDRLSTVVAGTYQPSEEYDY, SEQ ID NOs: 1-2

**C. DOCUMENTS CONSIDERED TO BE RELEVANT**

| Category* | Citation of document, with indication, where appropriate, of the relevant passages | Relevant to claim No. |
| --- | --- | --- |
| A | CN 113507937 A (ALETA BIOTHERAPEUTICS INC.) 15 October 2021 (2021-10-15) claims 1-21 | 1-19 |
| A | CN 114127115 A (INHIBRX, INC.) 01 March 2022 (2022-03-01) claims 1-42 | 1-19 |
| A | CN 114502586 A (2SEVENTY BIO, INC. et al.) 13 May 2022 (2022-05-13) claims 1-94 | 1-19 |
| A | JIANG, Yingping et al. "CLT030, a Leukemic Stem Cell-Targeting CLL1 Antibody-Drug Conjugate for Treatment of Acute Myeloid Leukemia" *Blood Advances*, Vol. 2, No. (14), 24 July 2018 (2018-07-24), 1738-1749 pp. 1738-1748 | 1-19 |

☑ Further documents are listed in the continuation of Box C.    ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **04 September 2023** | **11 September 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 635 981 A1**

## INTERNATIONAL SEARCH REPORT

International application No.

**PCT/CN2022/143892**

### C.    DOCUMENTS CONSIDERED TO BE RELEVANT

| Category* | Citation of document, with indication, where appropriate, of the relevant passages | Relevant to claim No. |
|---|---|---|
| A | ZHENG, Bing et al. "An Anti-CLL-1 Antibody-Drug Conjugate for the Treatment of Acute Myeloid Leukemia" <br> *Clinical Cancer Research*, Vol. 25, No. (4), 29 June 2018 (2018-06-29), 1358-1368 <br> pp. 1358-1366 | 1-19 |
| A | ZHANG, Hui et al. "Anti-CLL1 Chimeric Antigen Receptor T-Cell Therapy in Children with Relapsed/Refractory Acute Myeloid Leukemia" <br> *Clinical Cancer Research*, Vol. 27, No. (13), 08 April 2021 (2021-04-08), 3549-3555 <br> pp. 3549-3554 | 1-19 |

Form PCT/ISA/210 (second sheet) (July 2022)

24

## INTERNATIONAL SEARCH REPORT

| International application No. |
|---|
| **PCT/CN2022/143892** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/CN2022/143892**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **7-9 (in part), 11-16**
   because they relate to subject matter not required to be searched by this Authority, namely:

   Claims 7-9 (in part) and 11-16 relate to the use of a single-domain antibody or a biological material in the prevention or treatment of tumors, and in the prevention or treatment, screening, diagnosis or auxiliary diagnosis of CLL1 target-related diseases and a method, that is, claims 7-9 (in part) and 11-16 relate to subject matter for which no search is required by the International Searching Authority as defined in PCT Rule 39.1: (iv) methods for treatment of the human or animal body by surgery or therapy, as well as diagnostic methods. The international search is made on the basis of the use of a single-domain antibody or a biological material in the preparation of a drug for preventing or treating tumors, and for screening, diagnosing or assisting in diagnosing, preventing or treating CLL1 target-related diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

26

**INTERNATIONAL SEARCH REPORT**
Information on patent family members

International application No.

**PCT/CN2022/143892**

| Patent document cited in search report | | | Publication date (day/month/year) | Patent family member(s) | | | Publication date (day/month/year) |
|---|---|---|---|---|---|---|---|
| CN | 113507937 | A | 15 October 2021 | IL | 283476 | A | 29 July 2021 |
| | | | | KR | 20210124201 | A | 14 October 2021 |
| | | | | JP | 2022511799 | A | 01 February 2022 |
| | | | | EP | 3886903 | A1 | 06 October 2021 |
| | | | | EP | 3886903 | A4 | 13 July 2022 |
| | | | | CA | 3121478 | A1 | 04 June 2020 |
| | | | | MX | 2021006362 | A | 13 October 2021 |
| | | | | WO | 2020113063 | A1 | 04 June 2020 |
| | | | | US | 2022112292 | A1 | 14 April 2022 |
| | | | | MA | 54312 | A | 09 March 2022 |
| | | | | AU | 2019390483 | A1 | 15 July 2021 |
| CN | 114127115 | A | 01 March 2022 | CA | 3138972 | A1 | 12 November 2020 |
| | | | | MX | 2021013417 | A | 10 December 2021 |
| | | | | JP | 2022531306 | A | 06 July 2022 |
| | | | | WO | 2020227073 | A1 | 12 November 2020 |
| | | | | US | 2020347139 | A1 | 05 November 2020 |
| | | | | AU | 2020267349 | A1 | 11 November 2021 |
| | | | | KR | 20220004751 | A | 11 January 2022 |
| | | | | EP | 3966243 | A1 | 16 March 2022 |
| | | | | IL | 287781 | A | 01 January 2022 |
| | | | | SG | 11202111731 | WA | 29 November 2021 |
| CN | 114502586 | A | 13 May 2022 | US | 2022323496 | A1 | 13 October 2022 |
| | | | | CA | 3148027 | A1 | 12 November 2020 |
| | | | | SG | 11202111927 | QA | 29 November 2021 |
| | | | | IL | 287769 | A | 01 January 2022 |
| | | | | AU | 2020267639 | A1 | 06 January 2022 |
| | | | | BR | 112021022365 | A2 | 18 January 2022 |
| | | | | MA | 55905 | A | 16 March 2022 |
| | | | | EP | 3966249 | A1 | 16 March 2022 |
| | | | | EP | 3966249 | A4 | 10 May 2023 |
| | | | | MX | 2021013590 | A | 11 February 2022 |
| | | | | WO | 2020227475 | A1 | 12 November 2020 |
| | | | | KR | 20220031549 | A | 11 March 2022 |
| | | | | JP | 2022532556 | A | 15 July 2022 |

Form PCT/ISA/210 (patent family annex) (July 2022)